# EUROPEAN PATENT SPECIFICATION

(11) **EP 2 022 449 B1**
(45) Date of publication and mention of the grant of the patent: **27.03.2013**
(21) Application number: 08007515.3
(22) Date of filing: 10.11.2004
(51) Int. Cl.: A61F 2/46

(54) **Inserter for minimally invasive joint surgery**
Inserter für die MIV-Gelenkchirurgie
Porte-prothèse pour chirurgie d'articulation invasive minimale

(30) Priority: 10.11.2003 US 518768 P; 26.02.2004 US 548542 P; 10.04.2004 US 561141 P
(43) Date of publication of application: 11.02.2009
(62) Divisional of application: 04798816.7
(73) Proprietor: Greatbatch Medical SA, 2534 Orvin (CH)
(72) Inventor: Lechot, André, 2534 Orvin (CH); Desarzens, Yves, 2606 Corgémont (CH)
(74) Representative: Koepe, Gerd L.

(56) References cited:
- US-A- 5 741 289
- US-A1- 2002 151 931

## Description

### Background of the Invention

This invention relates to surgical inserters for aiding in installing orthopedic prostheses, and, more particularly, to easily sterilizable inserters for installing acetabular implants in the acetabular socket.

Complicated mechanical devices have crevices and recesses that are difficult, if not almost impossible to clean with ease. Devices that are not properly cleaned and sterilized run the risk of disease transfer from patient to patient following the emergence of certain "prions" that are not killed by normal hospital sterilisation and need to be physically removed by washing / rinsing.

Further, in surgical procedures in which access to the treatment site is limited, it is difficult to use current solutions without subjecting the patient to repeated abrasion and tissue trauma when inserting and extracting surgical instruments.

Further, the insertion of the implant is often problematic, and the orientation of the implant, particularly any fixing holes that might be pre-drilled in the implant is often critical to minimize recovery time of the patient. Still further, once the appropriate position of the implant is selected, it is often difficult to ensure that the position does not change upon insertion of the assembly through the incision.

What is needed therefore is an inserter that is easily adjustable, disassemblable, and cleanable. Further, what is needed is an inserter that enables the surgeon to better maneuver position and install an implant in a particular angular orientation.

US-A-5 741 289 discloses a surgical instrument having a housing and pivotable, removable component. When the component is unlocked it is pivotable in the housing. The component can be removed from the housing for cleaning purposes. Both, the housing and the pivotable, removable component are relatively straight. The locking mechanism for the pivotable, removable component includes pins and a top wall bump. However, the straight, pivotable, removable component pivots at or near the proximal end (a.k.a., the handle which is the non-surgical end). That pivot position is appropriate for straight, pivotable, removable components.

That pivot position means the straight, pivotable, removable component can have no bend therein. With no bend in the pivotable, removable component, US-A-5 741 289 does not have to be concerned about small radius internal corners, crevices, small gaps, and blind holes that are present in surgical devices that require bent medical instruments.

US 2002/151931 A1 discloses a surgical instrument with a main part and at least one movable part relative thereto which is also a straight device. A locking device is provided in which, in a first position, the movable part is arranged in an initial position, and in a second position, the movable part can be changed over into a cleaning position from a straight device.

### Summary of the Invention

The invention is related to a surgical inserter as defined in claim 1. The before mentioned objective is achieved by the features identified in the characterizing part of claim 1, which are considered to be the features distinguishing the current claimed invention from the technical solution proposed in US-A-5 741 289.

An acetabular inserter aids a surgeon in controlling the installation of an acetabular cup prosthesis generally having a central, female aperture. The inserter has a housing which encloses a drive train having, at a far end, a prosthesis engaging interface (e.g., a thread), and at the opposite end, a handle which facilitates turning of the drive train by the operator.

The inserter enables easy orientation of a prosthesis attached to its end, which is important because the prosthesis often has pre-drilled holes and thus, these must be properly positioned prior to fastening through these holes.

An objective of the invention is to be "easily cleaned" by quick and modular disassembly which enables access to all surfaces that they can be cleaned, the reduction in number of small radius internal corners, crevices and small gaps and the absence of blind holes.

Another object of the invention is to provide an inserter which enables the implant to be locked in an angular orientation prior to installation of the implant.

Another object of the invention is to provide a dual mechanism that uses common components to lock the implant in place as well as to provide for easy disassembly for cleaning and sterilization.

Another object of the invention is to minimise the number of pieces and the risk that parts could be lost.

### Brief Description of the Drawings

The attached drawings represent, by way of example, different embodiments of the subject of the invention.
FIG. **1A** is a cross-sectional side view of the impactor of the invention.
FIG. **1B** is a side view of the impactor of the invention.
FIG. **1C** is a perpective view of the impactor of the invention showing a one way catch mechanism.
FIG. **2A** is an operational side view of the impactor of the invention.
FIG. **2B** is an operational back view of the impactor of the invention.
FIG. **3A** is a perspective view of the impactor of the invention, showing a step of disassembly for cleaning.
FIG. **3B** is a perspective view of the impactor of the invention, showing another step of disassembly for cleaning.
FIG. **6A** is a side, cross sectional view of an alternate embodiment of the inserter of the invention.
FIG. **6B** is a perspective view of the jaws of the inserter of the invention.
FIG. **7A** is a side, cross sectional view of the tip of the inserter of the invention.
FIG. **7B** is a perspective cross sectional view of the tip of the inserter of the invention.
FIG. **8** is a perspective view of an actuation screw of the inserter of the invention.
FIG. **9A** is a cross-sectional side view of another alternate embodiment of the inserter of the invention, taken along line A-A in FIG. **9****B.**
FIG. **9B** is top view of the invention.
FIG. **10A** is a perspective view of the invention.
FIG. **10B** is a closeup perspective view of the invention, showing the dual locking mechanism.
FIG. **10C** is a section view of area 10C in FIG. **9A****.**

### Detailed Description of the Preferred Embodiment(s)

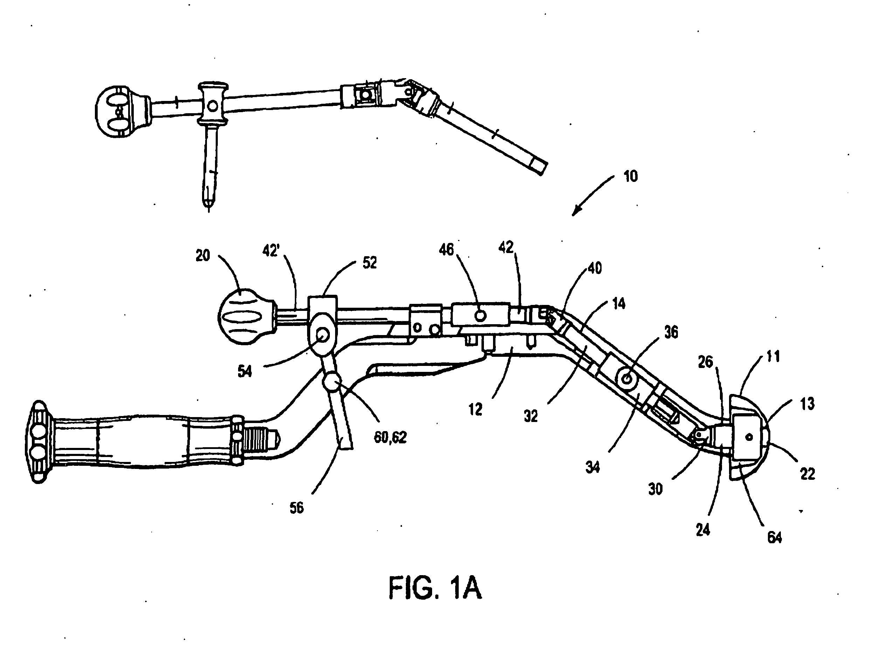

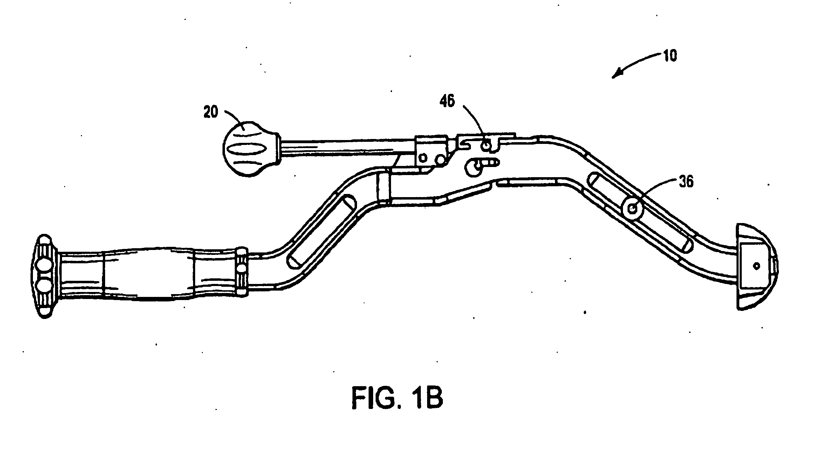

Referring now to FIGs. **1A-1C****,** an acetabular inserter 10 is provided to aid the surgeon in controlling the installation of an acetabular cup prosthesis 11 having a central, female aperture 13. The inserter 10 has a housing 12 which encloses a drive train 14 having, at a far end, a prosthesis engaging interface 16 (preferably threaded), and at the opposite end, a handle 20 which facilitates turning of the drive train by the operator. The housing 12 may be C-shaped, as shown, in order to minimize the invasiveness of the surgery by better clearing anatomical structures and tissue.

The interface 16 is cut on a boss 22 on a cylindrical piston 24 which slides in an axial hole 26 in the housing 12. The interface 16 is preferably threaded. The piston 24 is connected by way of a first U-joint 30 to a lever 32 which slides in a pivoting sleeve 34 fixed to the housing 12 via a pivot 36. The lever 32 is connected via a second U-joint 40 to a second pivoting lever 42 which is fixed to pivot in a catch 44 on a pivot pin 46. The catch 44 is essentially a pivot or a seat cut into the housing 12, against which the pivot pin 46 of the lever 42 is captured when a slide 50 is slid over the pin when engaged against the seat.

A slideable sleeve 52 slides over the lever 42 and has a trunion 54 to which a rod 56 is pivotally attached. The rod 56 passes through a one-way catch 60 in the housing 12. The one-way catch 60 can be a captured split wedge sleeve 62 having an inner diameter that just matches the outer diameter of the rod 56 and which is captured in a recess having a matching conical surface that surrounds the sleeve so as to allow the rod 56 to slide into the housing 12, but to prevent the rod from sliding out of the housing unless an unlock lever (not shown) is activated, such lever merely lifting the sleeve 62 out of engagement with the conical surface so as not to lock and to permit the rod to back out of the housing. Any number of alternative one way lock devices may be used however, the selection of which being within the skill of a person of ordinary skill in this field.

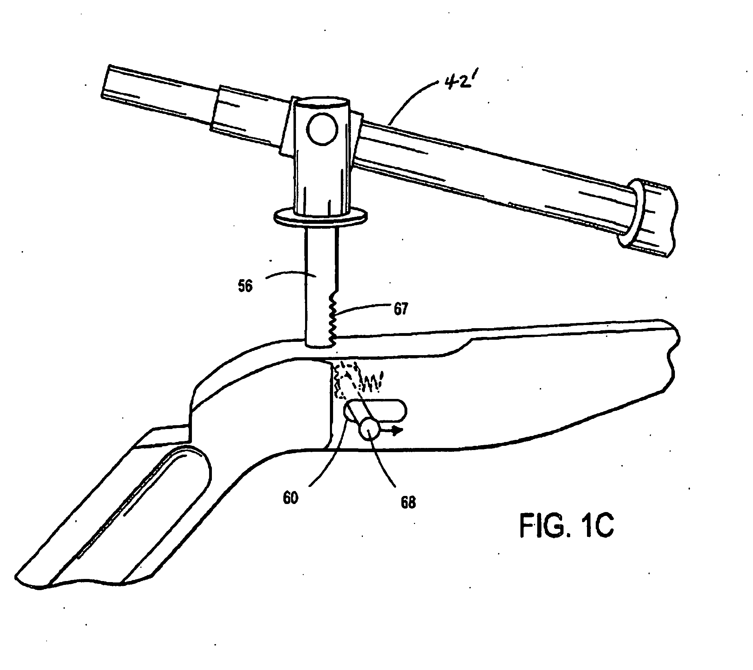

Referring now to FIG. **1C****,** an alternative embodiment of the one way catch mechanism 60 is shown. In this embodiment, the rod 56 passes through a one-way catch 60 in the housing 12. The one-way catch 60 has an inner recess that matches the outer diameter of the rod 56. The inner recess has a ratchet pawl (not shown) that locks against one way ratchet teeth 67 so as to allow the rod 56 to slide into the housing 12, but to prevent the rod from sliding out of the housing unless an unlock lever 68 is activated, such lever merely pulling the pawl away from the teeth to permit the rod to back out of the housing.

A polymeric impactor head 64 is molded over the end of the housing 12, to absorb the impact stresses incurred during use as an impactor. The head 64 is selected so as to have good frictional characteristics as well. Nevertheless, a metal, non-molded head may also be used with satisfactory results.

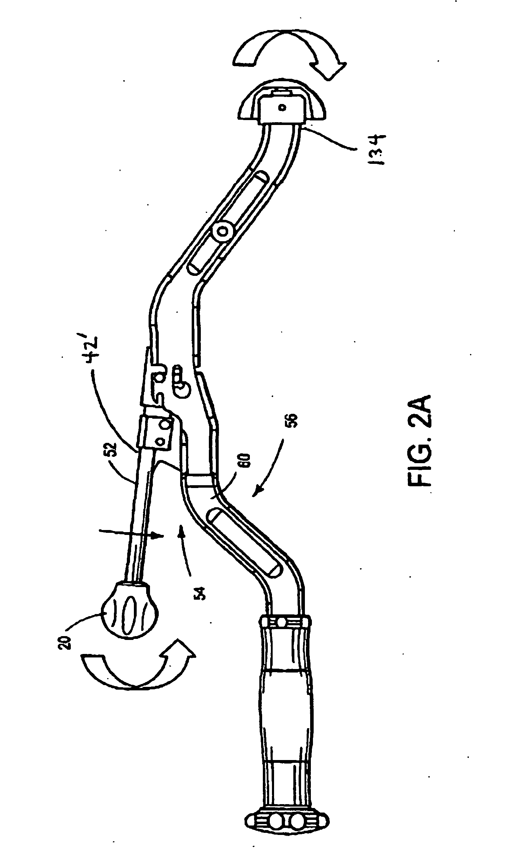

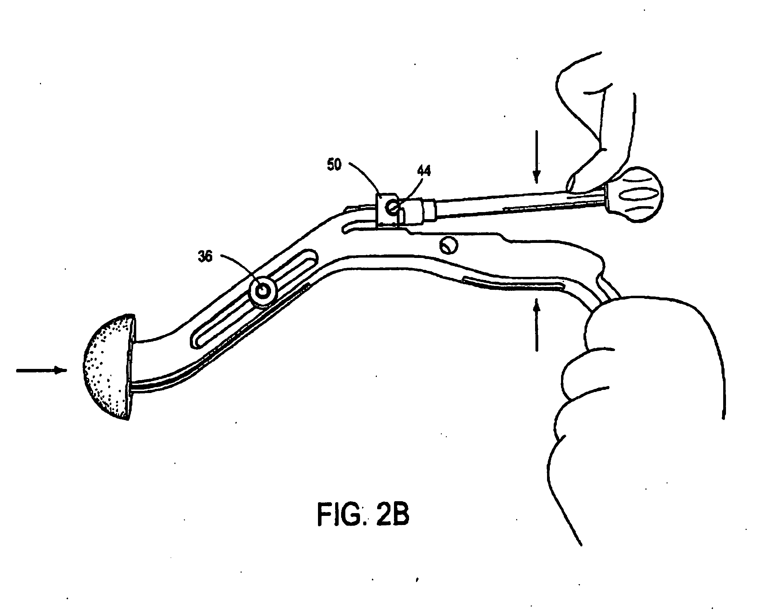

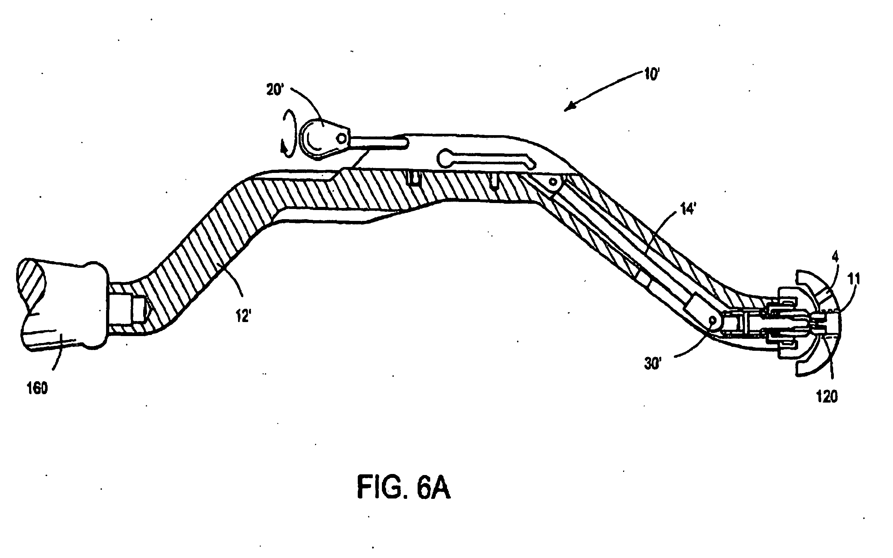

Referring now to FIGs. **2A - 2B****,** in operation, the interface 16 (preferably threaded) of the piston 24 is engaged with the hole 13 of the prosthesis 11. The operator may rotate the handle 20 about its axis to turn the drive train 14 in order to interface the piston 24 into the hole 13 or to orient the prosthesis in what he believes to be a correct or an initial position. Then, an end 42' of the lever 42 is urged downwardly toward the housing 12. Such downward movement acts through the drive train 14 to draw the piston 24 into the housing 12, and thus to cause the inner surface of the prosthesis 11 to be drawn against the head 64 so as to create a normal force between the inside of the prosthesis and the head so as to prevent rotation of the prosthesis 11 relative to the housing 12. The operator may use the one way locking mechanism 62 to lock the lever 42 in a position so as to lock the prosthesis 11 against the head 64, thus enabling the surgeon to pre-set and lock the position of the prosthesis prior to the installation thereof. Note that orientation of the prosthesis 11 is important because the prosthesis often has pre-drilled holes 4 (shown in **FIG. 6A**) and thus, these must be properly positioned prior to fastening through these holes.

The "easily cleaned" objective of the invention enables access to all surfaces that they can be cleaned (parts covering another part can be moved or removed to expose all surfaces), the reduction in number of small radius internal corners, crevices and small gaps and the absence of blind holes.

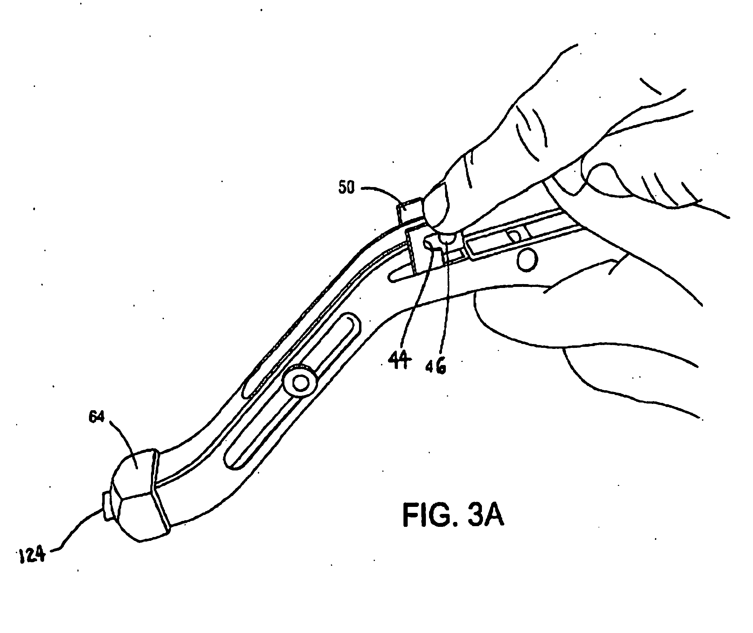

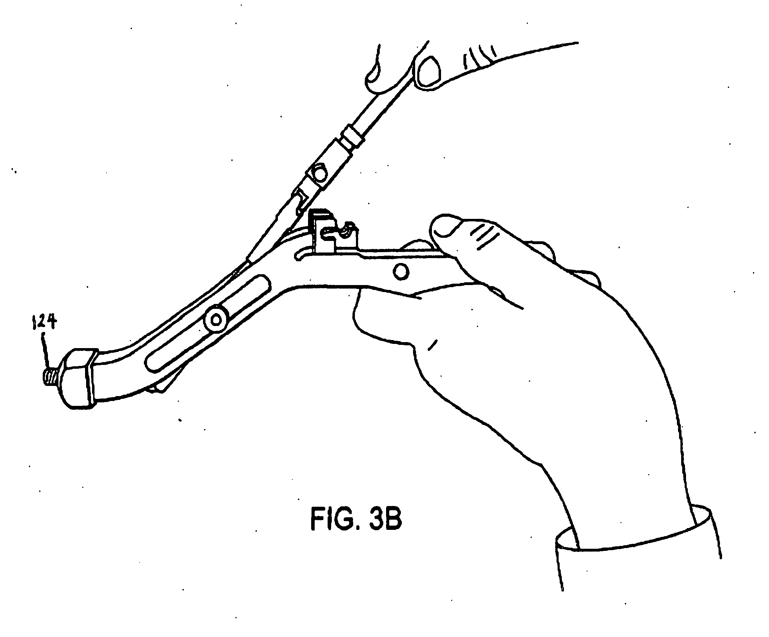

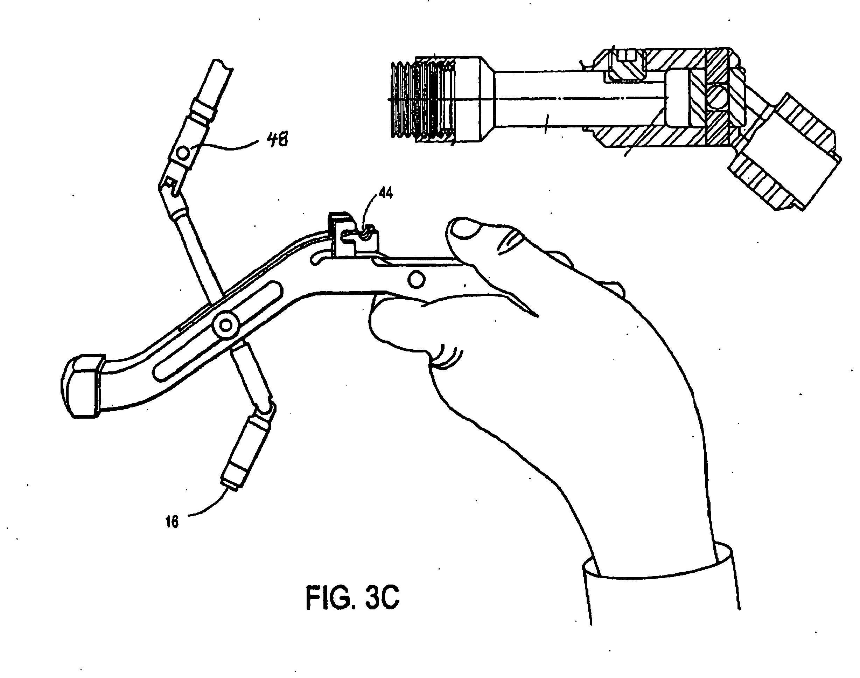

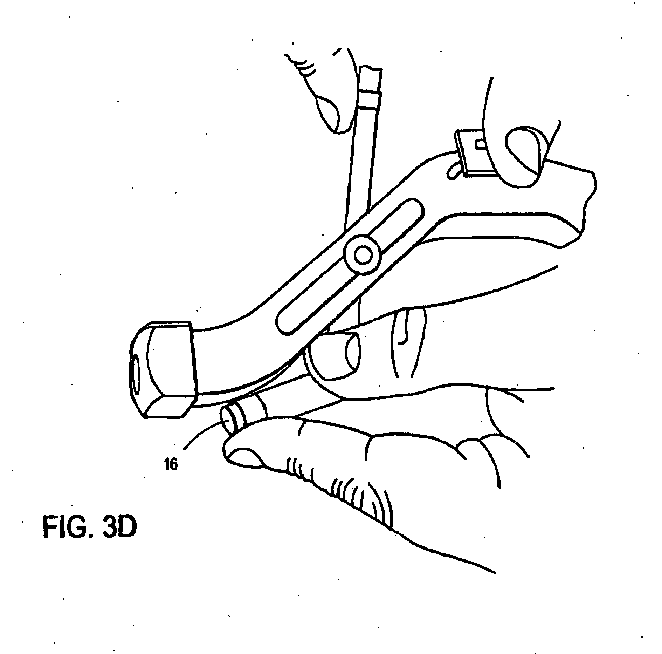

Referring now to FIGs. **3A** - **3D****,** in the embodiment shown, the device 10 is disassembled for cleaning by simply sliding the slide 50 back so as to release the pivot 46 and then lift the drive train 14 out of the housing but allow it to remain pivotally connected at pivot 36. As the drive train 14 is pivoted, the piston 16 is drawn out of the hole 26 in the housing 12. To reassemble after cleaning, the piston 16 is reinserted into the hole 26 and the drive train 14 is rotated back into position, with the one way locking mechanism entering its receiver and the pivot 46 again entering into the catch 44. The slide 50 is then slid over the pivot 46 and the inserter 10 is again ready for use.

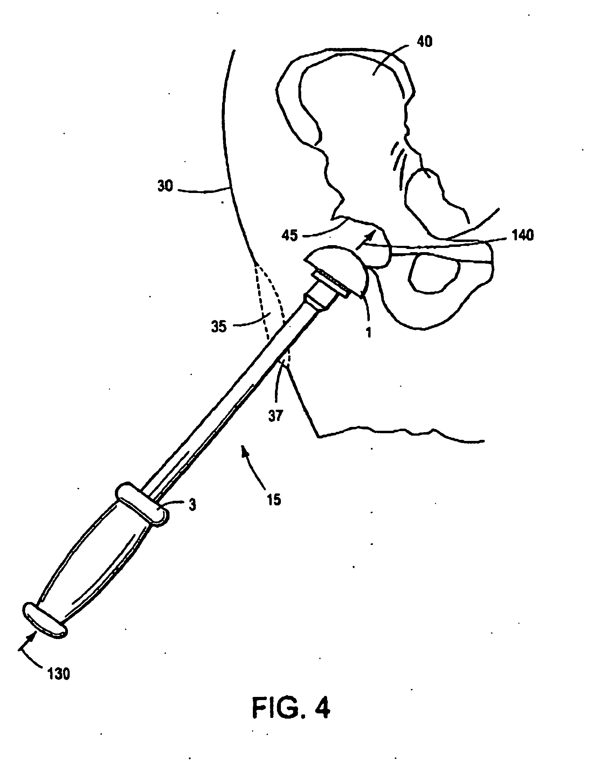

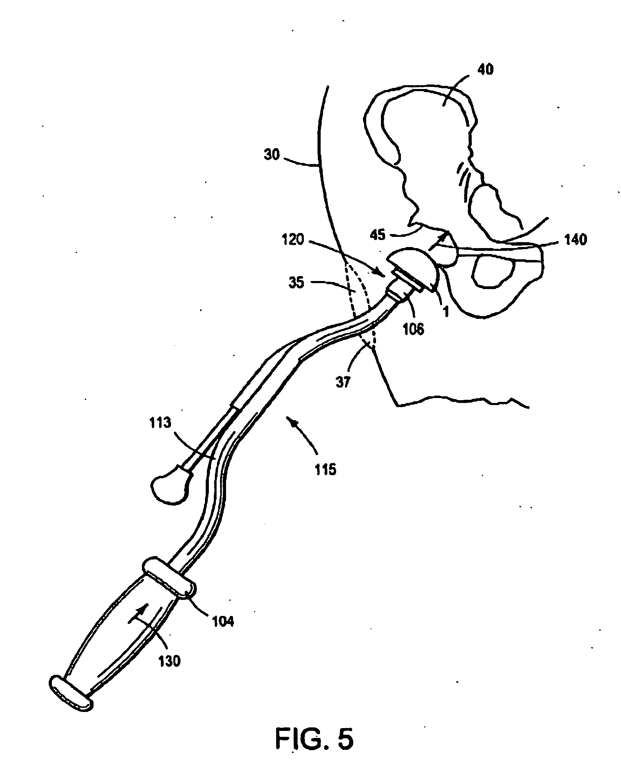

Referring to FIGS. **4-5****,** the inserter 115 and a prior art inserter 15, respectively, are shown passing through a miniature incision 35 in the patient's skin 30. In FIG. **4****,** the inserter 15 is shown approaching the acetabulum 40 in an orientation desirable to ream the socket 45. The difficulty with the prior art spindle 15 is shown as the shaft 3 impinges on the miniature incision 35 at edge of the incision 37. The current surgical protocols are being pushed to the limits and the incision sizes are being reduced in the hopes of increasing the patient's speed to recovery. In some cases surgeons are using a two-incision approach, one to reach the acetabulum and the other to reach the femur. Either one incision or two incision techniques demand less trauma to the patient requiring the instruments to be more optimally designed to make up for the lack of operating space. The reamer of FIG. **5** shows a new inserter 115, which has a bent housing 113 containing the drive shaft 107.

It is important to place the bends in the housing at critical locations to pass through the miniature incision without impinging on the skin 30 at 37 while still maintaining the same surgical protocol. The reason why the drive end 104 and the holding mechanism 120 need to be in line or on parallel axis is so that the applied force 130 results in an axial motion 140. This allows the surgeon to maintain the existing technique since inherently inserter 15 in FIG. **4** would give the same result since it has a straight drive shaft 3. This allows the surgeon to apply a load directly along the path of reaming.

It should be noted that a second head (not shown) can be mounted onto the front of the device 10, the head formed so as to conform with a surface of an acetabular cup liner, in order to enable the device to seat a liner as well as the cup.

The attached drawings represent, by way of example, different embodiments of the subject of the invention.

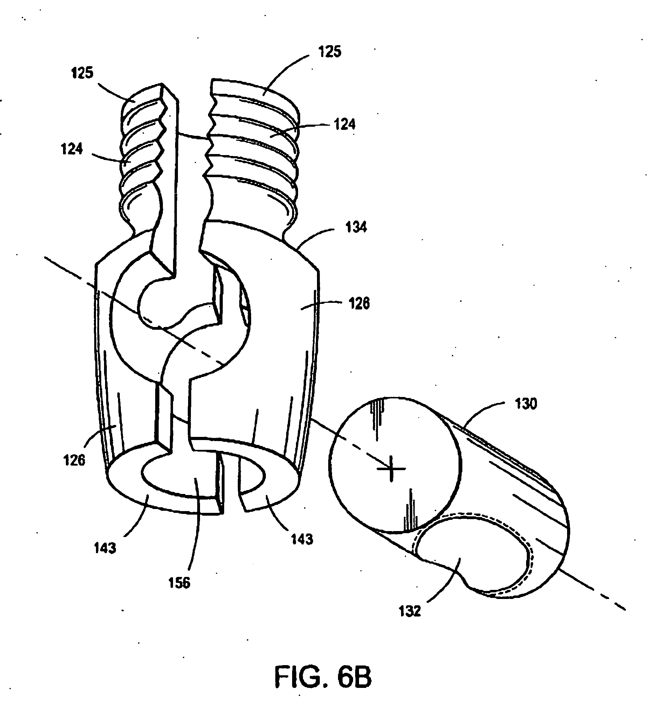

Referring now to FIG. **6A****,** in another embodiment, an acetabular inserter 10' is provided to aid the surgeon in controlling the installation of a hip prosthesis 11. The inserter 10' has a housing 12' which encloses a drive train 14' having, at a far end, a prosthesis engaging collet 120 (shown in detail in FIG. **6B****),** and at the opposite end, a knob or handle 20' which facilitates turning of the drive train by the operator. The housing 12' may be C-shaped, as shown, in order to minimize the invasiveness of the surgery by better clearing anatomical structures and tissue.

When the knob 20' is turned in one direction, the prosthesis-engaging collet 120 locks the prosthesis 11 against rotational movement. This enables the surgeon to pre-set and lock the position of the prosthesis 11 prior to the installation thereof. Such selective locking of the prosthesis 11 is important because the prosthesis 11 often has pre-drilled holes 4 and thus, these must be properly positioned prior to fastening through these holes. Further, the collet action of the collet 120 eliminates the need of threading the acetabular prosthesis 11 onto the end of the inserter 10' as the prosthesis can simply be placed over the collet and the collet expanded so as to grip internal threads 122 of the prosthesis 11. Note that to improve likelihood of alignment, the threads 124 on the jaws 126 may be replaced with longitudinally aligned dimples (not shown) having a profile that resembles threads but yet which minimizes the need for precise orientation of the internal threads of the prosthesis and the dimples on the jaws 126 of the collet 120 .

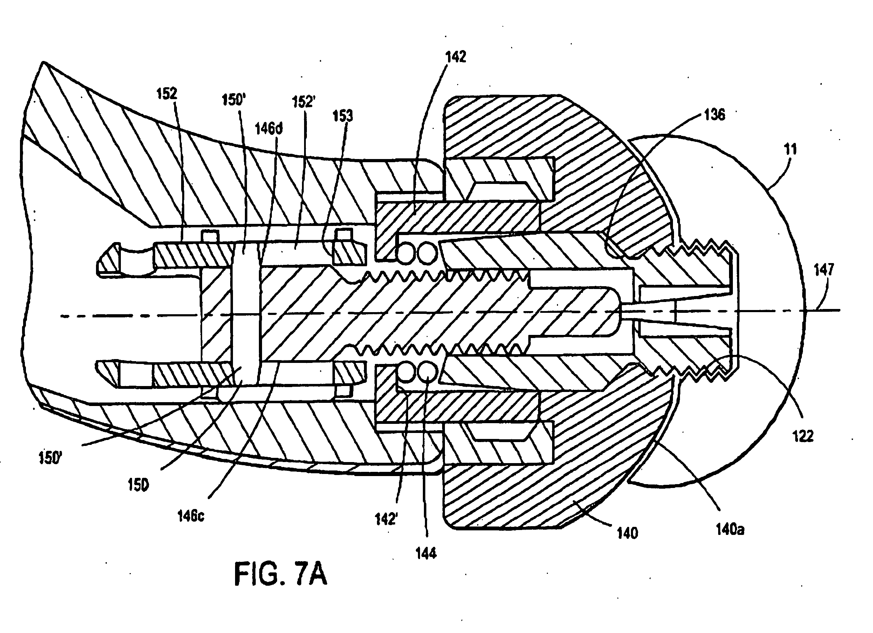

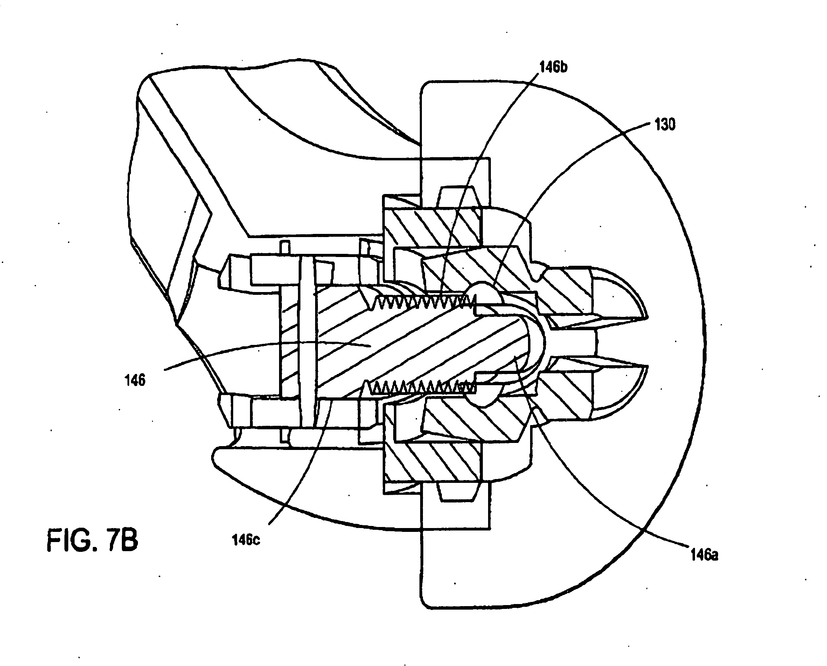

Referring now to FIGs. **6B****,** **7A****,** and **7B****,** the prosthesis-engaging collet 120 is made up of two jaws 126 which pivot on a ball or cylindrical pivot 130 having an threaded bore 34 passing transversely therethrough. The jaws 126 are constrained at one end 30' by a shoulder 136 of a plastic impaction head 140, along their outer circumference by a cup-shaped sleeve 142, and at an opposite end 143 by a compression spring 144 which reacts against a bottom end 142' of the sleeve 142.

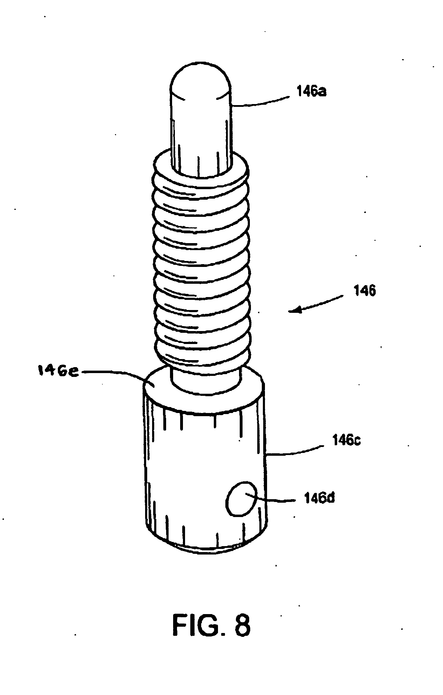

Referring as well to FIG. **8****,** an actuation screw 146 has a narrow cylindrical tip 146a, a threaded body 146b and a cylindrical interface 146c. The interface 146c has a cross hole 146d formed transversely to the longitudinal axis 147 of the screw 146. A U-joint shackle or clevis 152 slides over the interface 146c. A cross pin 150 passing through slots 152' in the shackle 152 and through the cross hole 146d of the interface 146c so that ends 150' of the cross pin protrude beyond the outermost cylindrical surface of the interface into the slots 152'.

The shackle 152 is part of a u-joint 30' of the drive train 14' which is connected to the knob 20' at an opposite end thereof. When the knob 20' is turned by the operator, the drive train 14' causes the actuation screw 146 to progress forward, and thus the tip 146a is able to move into a space 156 between the jaws 126 so as to progressively open the jaws until they are fully locked open. If the drive train 14' is turned further, then the ends 150' of the cross pin 150 contact an end 153 of the slot 152'at which point further turning causes the collet 120 to draw inward, pulling the prosthesis 11 into snug contact with the face of the impaction head 140. When fully open, the space 156 formed between the jaws 126 just receives the tip 146a and thus has substantially cylindrical boundaries. The external surface of the prosthesis-engaging end of the jaws 126 may take any suitable form so as to help lock the prosthesis 11 in place. A simple form which is effective may be a simple thread-matching profile which could include a pitch or not, depending on whether it is desired that the prosthesis be able to be removed by unthreading, despite the collet 120 being in a substantially locked position.

In operation, the prosthesis first is placed over or threaded end onto the collet 120 via a threaded hole 122. In a second step, the prosthesis 11 is oriented with respect to the form of the inserter 10', in order to minimally impact soft tissue. In a third step, the handle 160 of the inserter 10' is gripped and the prosthesis 11 placed through the incision 35. In a fourth step, the inserter 10' is used to impact the prosthesis 11 in place by impacting a rear portion of the inserter with a mallet, for example. Optionally, with the current design, it is envisioned that the prosthesis 11 be inserted into the incision 35 as a first step, potentially taking advantage of being able to more freely maneuver it into the incision and roughly position it prior to inserting the collet 120 of the inserter 10' into a mating hole-this optional procedure is used, the knob 20' of the inserter may then be turned by the operator to actuate the opening of the collet 120 and thus the fixing on the end of the inserter 10'. These optional steps substitute for the above mentioned four steps. In a fifth step, the knob 20' is turned in an opposite direction in order to release the prosthesis 11. In a final step, the inserter 10' is removed from the incision 35.

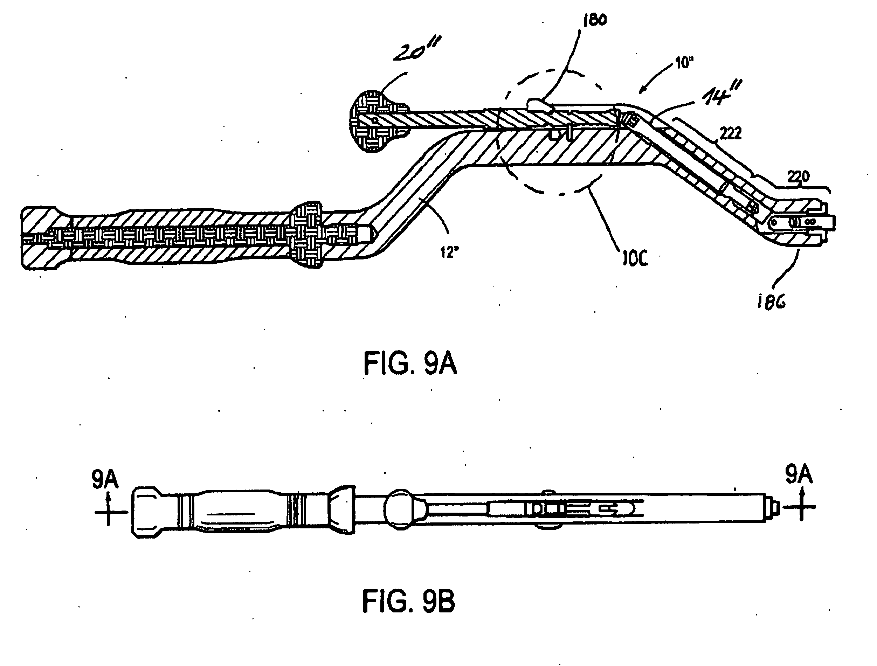

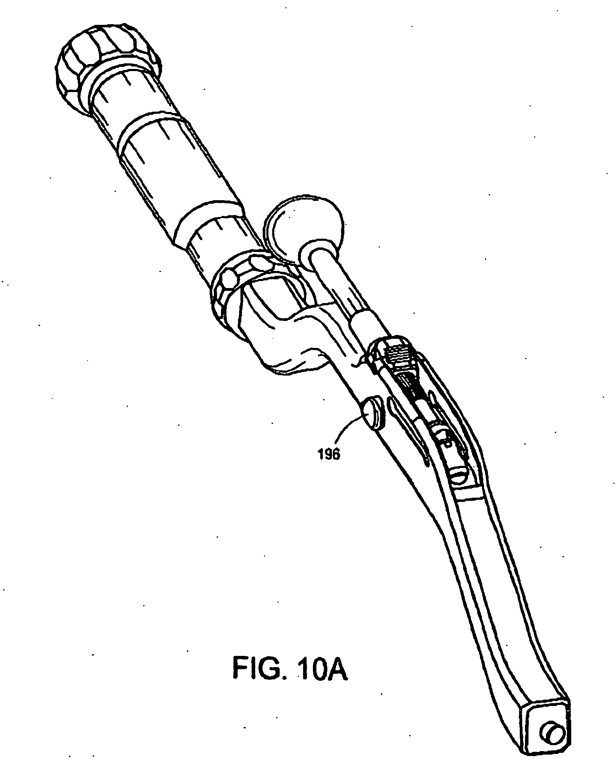

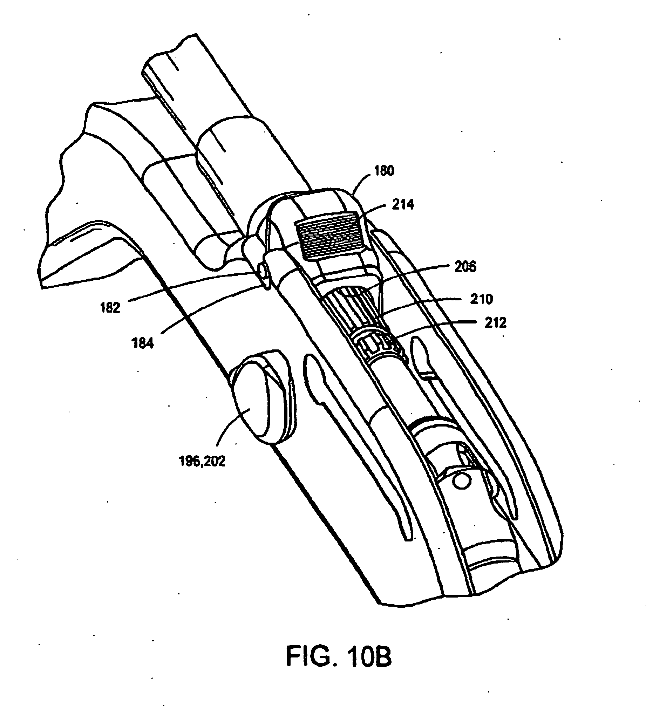

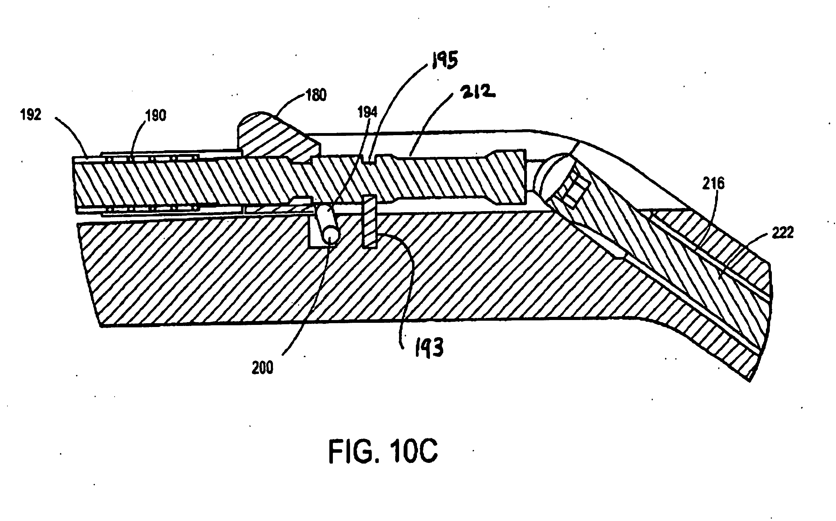

Referring now to FIGs. **9A - 9B****,** and **10A -** 10C, another embodiment of the acetabular inserter 10' is provided to aid the surgeon in controlling the installation of a hip prosthesis 11. The inserter 10" has a housing 12" which encloses a drive train 14" having, at a far end, a prosthesis engaging thread 124, and at the opposite end, a handle 20" which facilitates turning of the drive train by the operator. The housing 12" may be C-shaped, as shown, in order to minimize the invasiveness of the surgery by better clearing anatomical structures and tissue. A dual purpose locking mechanism, best shown in FIGs. **10B** and **10C****,** is made up of a latch housing 180 which is constrained against rotation by a key 182 and slot 184 and urged part-way into the slot 184 toward the coupling end 186 of the inserter 10" by a spring 190 captured between the latch housing 180 and a shaft 192 of the drive train 14". A retainer 193 enters a slot 195 in the component 200 in order to prevent relative axial movement of the drive train 14" when the latch housing 180 is in the locked position. The spring 190 urges the latch housing 180 against a cam stop 194 when a trigger 196 is positioned in a position so as to enable the drive train 14" to be turnable within the housing 10" by the operator rotating the handle 20". The cam stop 194 is connected to a shaft 200 to which an actuator component 202 is attached, to enable a user to turn the cam stop 196 in a position to block further movement of the latch housing 180 into the recess 184. When the cam stop 196 is turned so that it does not block further entry of the latch housing 180 into the recess 184, catches or teeth 206 inside the latch housing 180 are urged into engagement with serrations 26 cut into the outer circumference of the component 212 of the drive train 14". Because the latch housing 180 is constrained against rotational movement, the engagement of the catches 206 into the serrations 210 locks the drive train against rotational movement. This enables the surgeon to pre-set and lock the position of the prosthesis 11 prior to the installation thereof. Note that orientation of the prosthesis 11 is important because the prosthesis often has pre-drilled holes 4 and thus, these must be properly positioned prior to fastening through these holes.

The "easily cleaned" objective of the invention 10, 10', 10" enables access to all surfaces that they can be cleaned (parts covering another part can be moved or removed to expose all surfaces), the reduction in number of small radius internal corners, crevices and small gaps and the absence of blind holes.

In the embodiment shown, the device 10" is disassembled for cleaning by simply urging back on the latch housing 180 against the action of the spring 190 using a knurled surface 214 designed for that purpose. The latch housing 180 is urged back until a cross pin 182 is cleared of the recess 184 so that the drive train 14" can be pivoted away from the housing 12" so as to be approximately aligned with a central shaft 216 of the drive train. In the approximately aligned position, a slide-fit connection between the forward and rearward assemblies 220, 222 of the drive train 14" are separable, thus enabling essentially the entire rearward assembly of the drive train to be removed from the housing 12" for cleaning. Note the drive train 14" is immobilized during this urging of the latch housing 180 backwards due to the angled orientation of one component of the drive train about a corner or bend in the drive train.

In an advantage, the inserter 10' is simple and easy to use, without complex and possibly confusing locks activated with the thumb.

In another advantage, it is simple to select a desired orientation of the prosthesis 11.

In another advantage, due to the drawing of the prothesis 2 against the impaction head 40, the connection between the prosthesis 11. is robust as the connection is made without any play or gaps therebetween, ensuring good support during impaction.

An objective is to provide an inserter 10, 10', 10" that is easy to disassemble and for which the disassembly is easy to learn.

Another object of the invention is to provide a dual mechanism that uses common components to lock the implant in place as well as to provide for easy disassembly for cleaning and sterilization.

Another object of the invention is to minimise the number of pieces and the risk that parts could be lost.

The object of the invention is to provide an inserter 10, 10', 10" which enables the implant to be locked in an angular orientation prior to installation of the implant.

Multiple variations and modifications are possible in the embodiments of the invention described here. Although certain illustrative embodiments of the invention have been shown and described here, a wide range of modifications, changes, and substitutions is contemplated in the foregoing disclosure. In some instances, some features of the present invention may be employed without a corresponding use of the other features. Accordingly, it is appropriate that the foregoing description be construed broadly and understood as being given by way of illustration and example only, the scope of the invention being limited only by the appended claims.

## Claims

1. A surgical inserter (10, 10', 10") comprising a housing (12, 12', 12") with a proximal grip (3, 104) and a linkage chamber for receiving a linkage, the linkage being fixed to the housing (12, 12', 12") by locking means, and being pinned at a point in the chamber such that when the locking means is released, the linkage is pivotable out of the chamber for cleaning, **characterized in that**
- the housing (12, 12', 12"), and thus the linkage chamber is a bent housing (113), wherein the bent housing (113) has a bend that interconnects a first shaft linkage chamber area in a first axis and a second shaft linkage area in a second, different axis;
- the linkage has a drive train (14, 14', 14") having a pivotable U-joint (40), a first shaft (32, 216) and a second shaft (42, 212), wherein the drive train (14, 14', 14") further has a prosthesis engaging interface (16) at a far end (134) and a handle (20, 20', 20") at the opposite end (42');
- the U-joint (40) is positioned at the bend and interconnects the first shaft's proximal end and the second shaft's distal end; and
- the locking means (44, 50, 52, 54, 56, 60, 62, 67, 68; 124, 130, 142, 146; 180, 193, 194, 195, 196, 200, 202, 206, 210, 212, 214) applies a direct locking force upon the second shaft (42, 212), not the first shaft (32, 216), of the drive train (14, 14', 14").

2. The inserter (10, 10', 10") of claim 1, wherein the linkage has a ratchet pawl at a linkage chamber end cooperating with a pivot pin (46) by which the linkage is pinned in the chamber, the ratchet pawl being in driven communication with a driven end of a rod (56), and the rod having a drive end in driven communication with an unlock lever (68) having first mating means at the linkage retainer end of the linkage.

3. The inserter (10,10', 10") of claim 1 or claim 2, wherein the housing encloses the drive train (14, 14', 14") having, at the far end (134), a prosthesis engaging thread (124), and at the opposite end (42'), the handle (20, 20', 20") which facilitates turning of the drive train by the operator; and wherein the locking means (44, 50, 52, 54, 56, 60, 62, 67, 68; 124, 130, 142, 146; 180, 193, 194, 195, 196, 200, 202, 206, 210, 212, 14) is associated with the housing which selectively locks the drive train, and thus a prosthesis, in position.

4. The inserter (10, 10', 10") of claim 3, wherein further the opposite end (42') of the drive train has a latch device (52, 54, 56, 60, 62; 44, 50; 180) which enables quick removal from the housing for cleaning and sterilization.

5. The inserter (10, 10', 10") of claim 3, wherein the drive train (14,14', 14") includes at least one U-joint (30') at a bend in the housing (12,12', 12").

6. The inserter (10, 10', 10") of claim 3, wherein the housing (12, 12', 12") is C-shaped.

7. The inserter (10) of claim 3, wherein the locking mechanism (44, 50, 52, 54, 56, 60, 62, 67, 68) comprises the drive train (14) having a threaded, prosthesis engaging tip (146a), the drive train further including the second shaft (42, 212) which is disposed in the housing (12) so as to rotate on the first shaft (32, 216) like a fulcrum, such that, actuation of the second shaft (42, 212) draws the threaded tip (146a) into the housing and, when connected to a prosthesis (11), draws the prosthesis against an impaction surface (140a), wherein sufficient friction may be generated therebetween to lock the prosthesis in place.

8. The inserter (10) of claim 5, wherein the second shaft (42) has the handle (20) attached to its extreme end, the handle enabling a user to orient the tip (146a).

9. The inserter (10) of any one of claims 3 to 8, wherein a lockable, variable length link (56) is attached between the second shaft (42, 212) and the housing (12) in order to permit a user to vary pressure that the tip can exert against the impaction head (140).

10. The inserter (10) of claim 9, wherein the rod (56) is infinitely variable and unlockable via the unlock lever (68) in order to permit release of pressure on the prosthesis (11).

11. The inserter (10) of claim 10, wherein the prosthesis engaging tip (146a) is connected by way of a first U-joint (30') to the first shaft (32) which slides in a pivoting sleeve (34) fixed to the housing via a pivot (36).

12. The inserter (10) of claim 3, wherein a one-way ratchet teeth (67) prevents the rod (56) connected to the second shaft (42) from sliding out of the housing (12) unless an unlock lever (68) is activated.

13. The inserter (10') of claim 3, wherein the locking mechanism (124,130, 142, 146) is an expandable collet (120) which expands when a knob (20'), adjacent the handle (60), turned in one direction so as to lock the collet (120) against a surface of a prosthesis (11) in order to prevent the prosthesis from rotation, thus enabling the surgeon to pre-set and lock the position of the prosthesis, prior to the installation thereof.

14. The inserter (10') of claim 13, wherein the collet (120) is comprised of two jaws (124) having opposite ends (125,126) pivoting on the first shaft (32), one end of which being adapted to engage an interior surface of a prosthesis (11), the prosthesis engaging ends being drawn away from one another when an actuator piston (146), which passes through a ball or cylindrical pivot (130), is drawn therebetween, thereby eliminating the need of threading the acetabular prosthesis (11) onto the tip (125) of the inserter as the prosthesis can simply be placed over the collet and the collet expanded so as to grip the internal threads (122) of the prosthesis.

15. The inserter (10") of claim 3, wherein further, the locking means (180, 193, 194, 195, 196, 200, 202, 206, 210. 212) is made up of a latch housing (180) which is constrainable against rotation while being urged part-way into a recess (184) toward the engagement end (186) of a head (140) by a spring (190) captured between the latch housing (180) and a shaft (212) of the drive train, the spring urging the latch housing against a cam stop (194) when a trigger (196, 200) is positioned so as to selectively:
(i) enable the drive train to be turnable within the housing (12") by the operator rotating the handle (160), the cam stop (194) being connected to a shaft (200) to which an actuator component (196) is attached,
(ii) enable a user to turn the cam stop (194) in a position to block further movement of the latch housing (180) into the recess (184), such that when the cam stop is turned so that it does not block further entry of the latch housing into the recess (184), catches (206) inside the latch housing are urged into engagement with serrations (210) cut into the outer circumference of a component (212) of the drive train, wherein the engagement of the catches (206) into the serrations (210) constrains the latch housing (190) against rotational movement and locks the drive train (14") against rotational movement, the selectivity enabling the surgeon to pre-set and lock the position of the prosthesis (11) prior to the installation thereof, wherein the latch housing (180) may be unlatched from the housing so as to enable quick and modular disassembly and that the inserter is easily cleanable.

## Patentansprüche

1. Chirurgischer Inserter (10, 10', 10") mit einem Gehäuse (12, 12', 12") mit einem proximalen Griff (3, 104) und einer Verbindungskammer zur Aufnahme einer Verbindung, wobei die Verbindung mit dem Gehäuse (12, 12', 12") durch ein Arretierungselement befestigt ist, und an einem Punkt in der Kammer fixiert ist, so dass, wenn das Arretierungselement freigegeben wird, die Verbindung aus der Kammer zur Reinigung schwenkbar ist, **dadurch gekennzeichnet, dass**
- das Gehäuse (12, 12', 12"), und folglich die Verbindungskammer, ein gebogenes Gehäuse (113) ist, wobei das gebogene Gehäuse (113) eine Biegung aufweist, die einen ersten Verbindungskammer-Schaftbereich in einer ersten Achse und einen zweiten Verbindungs-Schaftbereich in einer zweiten, unterschiedlichen Achse miteinander verbindet;
- die Verbindung einen Antriebsmechanismus (14, 14', 14") aufweist, der ein drehbares Gelenkstück (40), einen ersten Schaft (32, 216) und einen zweiten Schaft (42, 212) aufweist, wobei der Antriebsmechanismus (14, 14', 14") weiter eine prothesenkontaktierende Schnittstelle (16) an einem fernen Ende (134) und einen Griff (20, 20', 20") an dem entgegengesetzten Ende (42') aufweist;
- das Gelenkstück (40) an der Biegung positioniert ist und das proximale Ende des ersten Schafts und das distale Ende des zweiten Schafts miteinander verbindet; und
- das Arretierungselement (44, 50, 52, 54, 56, 60, 62, 67, 68; 124, 130, 142, 146; 180, 193, 194, 195, 196, 200, 202, 206, 210, 212, 214) eine direkte Arretierungskraft auf den zweiten Schaft (42, 212), nicht auf den ersten Schaft (32, 216), des Antriebsmechanismus (14, 14', 14") ausübt.

2. Inserter (10, 10', 10") nach Anspruch 1, wobei die Verbindung eine Sperrklinke an einem Verbindungskammerende aufweist, die mit einem Drehzapfen (46) zusammenwirkt, mit dem die Verbindung in der Kammer fixiert ist, wobei die Sperrklinke in Antriebsverbindung mit einem angetriebenen Ende eines Stabs (56) ist, und der Stab ein Antriebsende aufweist, das in Antriebsverbindung mit einem Entriegelungshebel (68) steht, der ein erstes Anschlusselement an dem Verbindungs-Arretierstiftende der Verbindung aufweist.

3. Inserter (10, 10', 10") nach Anspruch 1 oder 2, wobei das Gehäuse den Antriebsmechanismus (14, 14', 14") umgibt, der an einem fernen Ende (134) ein protheseneingreifendes Gewinde (124) und an dem entgegengesetzten Ende (42') einen Griff (20, 20', 20") aufweist, der das Drehen des Antriebsmechanismus durch den Benutzer ermöglicht; und wobei die Arretierungseinrichtung (44, 50, 52, 54, 56, 60, 62, 67, 68; 124, 130, 142, 146; 180, 193, 194, 195, 196, 200, 202, 206, 210, 212, 214) mit dem Gehäuse verbunden ist, die selektiv den Antriebsmechanismus und damit die Prothese in der Position arretiert.

4. Inserter (10, 10', 10") nach Anspruch 3, wobei ferner das entgegengesetzte Ende (42') des Antriebsmechanismus eine Verriegelungsvorrichtung (52, 54, 56, 60, 62; 44, 50; 180) aufweist, die die schnelle Entnahme aus dem Gehäuse zur Reinigung und Sterilisation ermöglicht.

5. Inserter (10, 10', 10") nach Anspruch 3, wobei der Antriebsmechanismus (14, 14', 14") mindestens ein Gelenkstück (30') an einer Biegung in dem Gehäuse (12, 12', 12") aufweist.

6. Inserter (10, 10', 10") nach Anspruch 3, wobei das Gehäuse (12, 12`, 12") C-förmig ist.

7. Inserter (10) nach Anspruch 3, wobei der Arretierungsmechanismus (44, 50, 52, 54, 56, 60, 62, 67, 68) einen Antriebsmechanismus (14) mit einer mit einem Gewinde versehenen, prothesenkontaktierenden Spitze (146a) aufweist, wobei der Antriebsmechanismus weiter den zweiten Schaft (42, 212) aufweist, der in dem Gehäuse (12) angeordnet ist, um an dem ersten Schaft (32, 216) ähnlich einem Drehpunkt zu rotieren, so dass die Betätigung des zweiten Schafts (42, 212) die Gewindespitze (146a) in das Gehäuse zieht, und, wenn sie an einer Prothese (11) angeschlossen ist, die Prothese gegen eine Einschlagsoberfläche (140a) gezogen wird, wobei eine ausreichende Reibung dazwischen erzeugt werden kann, um die Prothese zu fixieren.

8. Inserter (10) nach Anspruch 5, wobei der zweite Schaft (42) einen Griff (20) aufweist, der an seinem äußeren Ende angebracht ist, wobei der Griff einen Benutzer befähigt, die Spitze (146a) auszurichten.

9. Inserter (10) nach einem der Ansprüche 3 bis 8, wobei ein arretierbarer Anschluss (56) mit variabler Länge zwischen dem zweiten Schaft (42, 212) und dem Gehäuse (12) angebracht ist, um einem Benutzer zu ermöglichen den Druck zu variieren, den die Spitze gegen den Einschlagkopf (140) ausüben kann.

10. Inserter (10) nach Anspruch 9, wobei der Stab (56) stufenlos veränderbar und über einen Entriegelungshebel (68) entsperrbar ist, um die Freisetzung von Druck auf die Prothese (11) zu ermöglichen.

11. Inserter (10) nach Anspruch 10, wobei die prothesenkontaktierende Spitze (146a) durch ein erstes Gelenkstück (30') mit dem ersten Schaft (32) verbunden ist, der in einer Schwenkhülse (34) gleitet, die über einen ersten Drehzapfen (36) an dem Gehäuse fixiert ist.

12. Inserter (10) nach Anspruch 3, wobei ein Einweg-Sperrmechanismus (67) ein Herausgleiten des Stabs (56), der mit dem zweiten Schaft (42) verbunden ist, aus dem Gehäuse (12) verhindert, sofern nicht ein Entriegelungshebel (68) aktiviert wird.

13. Inserter (10') nach Anspruch 3, wobei der Arretierungsmechanismus (124, 130, 142, 146) eine spreizbare Hülse (120) ist, die sich ausdehnt, wenn ein Knopf (20'), der neben dem Griff (160) liegt, in einer Richtung gedreht wird, so dass die Hülse (120) an einer Oberfläche einer Prothese (11) arretiert wird, um eine Drehung der Prothese zu verhindern, wodurch es dem Chirurgen ermöglicht wird, die Position der Prothese, vor der Installation davon, voreinzustellen und zu arretieren.

14. Inserter (10') nach Anspruch 13, wobei die Hülse (120) zwei Backen (124) mit gegenüberliegenden Enden (125, 126), die am ersten Schaft (32) schwenkbar sind, aufweist, wobei ein Ende für den Eingriff mit einer innere Oberfläche einer Prothese (11) geeignet ist, wobei die prothesenkontaktierenden Enden voneinander weggezogen sind, wenn ein Betätigungskolben (146), der durch einen kugel- oder zylinderförmigen Drehzapfen (130) verläuft, dazwischen gezogen wird, wodurch die Notwendigkeit, die Hüftgelenkpfannenprothese (11) auf der Spitze (125) des Inserters mit einem Gewinde zu versehen, beseitigt wird, da die Prothese einfach über der Hülse angeordnet werden kann und die Hülse sich ausdehnt, um das Innengewinde (122) der Prothese zu greifen.

15. Inserter (10") nach Anspruch 3, wobei ferner das Arretierungselement (180, 193, 194, 195, 196, 200, 202, 206, 210, 212) aus einem Verriegelungsgehäuse (180) aufgebaut ist, das gegen Drehung einschränkbar ist, solange es einen Teil des Weges in eine Ausnehmung (184), in Richtung auf das Kontaktende (186) eines Kopfs (140) durch eine Feder (190) gedrängt wird, die zwischen dem Verriegelungsgehäuse (180) und einem Schaft (212) des Antriebsmechanismus erfasst ist, wobei die Feder das Verriegelungsgehäuse gegen einen Anschlagnocken (194) drängt, wenn ein Auslöser (196, 200) positioniert ist, um selektiv:
i) dem Antriebsmechanismus zu ermöglichen, innerhalb des Gehäuses (12") drehbar zu sein, indem der Benutzer den Griff (160) dreht, wobei der Anschlagnocken (194) mit einem Schaft (200), an dem eine Betätigungskomponente (196) befestigt ist, verbunden ist,
ii) einem Benutzer zu ermöglichen, den Anschlagnocken (194) in eine Position zu drehen, um eine weitere Bewegung des Verriegelungsgehäuses (180) in die Ausnehmung (184) zu blockieren, so dass, wenn der Anschlagnocken gedreht wird, so dass er nicht das weitere Eindringen des Verriegelungsgehäuses in die Ausnehmung (184) blockiert, Rasten (206) innerhalb des Verriegelungsgehäuses in Eingriff mit Verzahnungen (210) gedrängt werden, die in den äußeren Umfang einer Komponente (212) des Antriebsmechanismus geschnitten sind, wobei der Eingriff der Rasten (206) in die Verzahnungen (210) das Verriegelungsgehäuse (180) gegen eine Rotationsbewegung einschränkt und den Antriebsmechanismus (14") gegen eine Rotationsbewegung arretiert, wobei die Selektivität dem Chirurgen ermöglicht, die Position der Prothese (11), vor der Installation davon, voreinzustellen und zu arretieren, wobei das Verriegelungsgehäuse (180) von dem Gehäuse entriegelt werden kann, um eine schnelle und modulare Demontage zu ermöglichen und dass der Inserter leicht zu reinigen ist.

## Revendications

1. Porte-prothèse (10, 10', 10") chirurgical comprenant un logement (12, 12', 12") avec un dispositif de préhension proximal (3, 104) et une chambre de liaison pour recevoir une liaison, la liaison étant fixée au logement (12, 12', 12") par un moyen de verrouillage, et étant tourillonnée en un point dans la chambre de telle sorte que, lorsque le moyen de verrouillage est libéré, la liaison peut pivoter hors de la chambre pour nettoyage, **caractérisé en ce que**
- le logement (12, 12', 12"), et ainsi la chambre de liaison, est un logement incurvé (113), dans lequel le logement incurvé (113) a une courbe qui interconnecte une zone de chambre de liaison de premier arbre dans un premier axe et une zone de liaison de deuxième arbre dans un deuxième axe différent ;
- la liaison a un train de transmission (14, 14', 14") ayant une articulation en U (40) pivotante, un premier arbre (32, 216) et un deuxième arbre (42, 212), dans lequel le train de transmission (14, 14', 14") a en outre une interface d'engagement de prothèse (16) au niveau d'une extrémité distante (134) et une poignée (20, 20', 20") au niveau de l'extrémité opposée (42') ;
- l'articulation en U (40) est positionnée au niveau de la courbure et interconnecte l'extrémité proximale du premier arbre et l'extrémité distale du deuxième arbre ; et
- le moyen de verrouillage (44, 50, 52, 54, 56, 60, 62, 67, 68 ; 124, 130, 142, 146 ; 180, 193, 194, 195, 196, 200, 202, 206, 210, 212, 214) applique une force de verrouillage directe sur le deuxième arbre (42, 212), non sur le premier arbre (32, 216), du train de transmission (14, 14', 14").

2. Porte-prothèse (10, 10', 10") selon la revendication 1, dans lequel la liaison a un cliquet d'arrêt au niveau d'une extrémité de chambre de liaison coopérant avec un tourillon (46) par lequel la liaison est tourillonnée dans la chambre, le cliquet d'arrêt étant en communication entraînée avec une extrémité entraînée d'une tige (56), et la tige ayant une extrémité d'entraînement en communication entraînée avec un levier de déverrouillage (68) ayant un premier moyen de mise en correspondance au niveau de l'extrémité de maintien de liaison de la liaison.

3. Porte-prothèse (10, 10', 10") selon la revendication 1 ou la revendication 2, dans lequel le logement renferme le train de transmission (14, 14', 14") ayant, au niveau de l'extrémité distante (134), un filetage d'engagement de prothèse (124), et à l'extrémité opposée (42'), la poignée (20, 20', 20") qui facilite la mise en rotation du train de transmission par l'opérateur ; et dans lequel le moyen de verrouillage (44, 50, 52, 54, 56, 60, 62, 67, 68 ; 124, 130, 142, 146 ; 180, 193, 194, 195, 196, 200, 202, 206, 210, 212, 214) est associé avec le logement qui verrouille sélectivement le train de transmission, et ainsi une prothèse, en position.

4. Porte-prothèse (10, 10', 10") selon la revendication 3, dans lequel, en outre, l'extrémité opposée (42') du train de transmission a un dispositif de verrou (52, 54, 56, 60, 62 ; 44, 50 ; 180) qui permet un enlèvement rapide du logement pour nettoyage et stérilisation.

5. Porte-prothèse (10, 10', 10") selon la revendication 3, dans lequel le train de transmission (14, 14', 14") inclut au moins une articulation en U (30') au niveau d'une courbure dans le logement (12, 12', 12").

6. Porte-prothèse (10, 10', 10") selon la revendication 3, dans lequel le logement (12, 12', 12") est en forme de C.

7. Porte-prothèse (10) selon la revendication 3, dans lequel le moyen de verrouillage (44, 50, 52, 54, 56, 60, 62, 67, 68) comprend le train de transmission (14) ayant un embout d'engagement de prothèse (146a) fileté, le train de transmission incluant en outre le deuxième arbre (42, 212) qui est disposé dans le logement (12) de façon à tourner sur le premier arbre (32, 216) comme un point d'appui, de telle sorte que l'actionnement du deuxième arbre (42, 212) tire l'embout (146a) fileté dans le logement et, lorsqu'il est connecté à une prothèse (11), tire la prothèse contre une surface d'inclusion (140a), dans lequel un frottement suffisant peut être généré entre celles-ci pour verrouiller la prothèse en place.

8. Porte-prothèse (10) selon la revendication 5, dans lequel le deuxième arbre (42) a la poignée (20) fixée à son extrémité ultime, la poignée permettant à un utilisateur d'orienter l'embout (146a).

9. Porte-prothèse (10) selon l'une quelconque des revendications 3 à 8, dans lequel une liaison (56) verrouillable de longueur variable est fixée entre le deuxième arbre (42, 212) et le logement (12) afin de permettre à un utilisateur de faire varier la pression que l'embout peut exercer contre la tête d'inclusion (140).

10. Porte-prothèse (10) selon la revendication 9, dans lequel la tige (56) est variable à l'infini et déverrouillable par l'intermédiaire du levier de déverrouillage (68) afin de permettre la libération de la pression sur la prothèse (11).

11. Porte-prothèse (10) selon la revendication 10, dans lequel l'embout d'engagement de prothèse (146a) est connecté au moyen d'une première articulation en U (30') au premier arbre (32) qui coulisse dans un manchon pivotant (34) fixé au logement par l'intermédiaire d'un pivot (36).

12. Porte-prothèse (10) selon la revendication 3, dans lequel des dents de cliquet unidirectionnel (67) empêchent la tige (56) connectée au deuxième arbre (42) de glisser hors du logement (12) si un levier de déverrouillage (68) n'est pas activé.

13. Porte-prothèse (10') selon la revendication 3, dans lequel le mécanisme de verrouillage (124, 130, 142, 146) est une extensible pince de serrage (120) que s'étend lorsque un bouton (20'), adjacente à la poignée (160), est tourné dans un sens de façon à verrouiller la pince de serrage (120) contre une surface d'une prothèse (11) afin d'empêcher la prothèse de tourner, permettant ainsi au chirurgien de prérégler et de verrouiller la position de la prothèse, avant l'installation de celle-ci.

14. Porte-prothèse (10') selon la revendication 13, dans lequel la pince de serrage (120) est composée de deux mâchoires (124) ayant des extrémités opposées (125, 126) pivotant sur le premier arbre (32), une extrémité de laquelle étant adaptée à engager une surface intérieure d'une prothèse (11), les extrémités d'engagement de prothèse étant écartées l'une de l'autre lorsqu'un piston d'actionneur (146), qui passe à travers une bille ou un pivot cylindrique (130), est tiré entre celles-ci, éliminant ainsi la nécessité de fixation par filetage de la prothèse (11) acétabulaire sur l'embout (125) du porte-prothèse puisque la prothèse peut être simplement placée sur la pince de serrage et la pince de serrage étendue de manière à agripper les filets internes (122) de la prothèse.

15. Porte-prothèse (10") selon la revendication 3, dans lequel, en outre, le moyen de verrouillage (180, 193, 194, 195, 196, 200, 202, 206, 210, 212) est constitué d'un logement de verrou (180) pouvant être contraint en rotation tout en étant forcé à mi-chemin à l'intérieur d'un renfoncement (184) vers l'extrémité d'engagement (186) d'une tête (140) par un ressort (190) pris entre le logement de verrou (180) et un arbre (212) du train de transmission, le ressort forçant le logement de verrou contre une butée de came (194) lorsqu'une gâchette (196, 200) est positionnée de manière à sélectivement :
(i) permettre la rotation du train de transmission à l'intérieur du logement (12") par l'opérateur en faisant tourner la poignée (160), la butée de came (194) étant connectée à un arbre (200) auquel un composant d'actionneur (196) est fixé,
(ii) permettre à un utilisateur de faire tourner la butée de came (194) dans une position de façon à bloquer tout mouvement ultérieur du logement de verrou (180) à l'intérieur du renfoncement (184), de telle sorte que, lorsque la butée de came est tournée de façon à ce qu'elle ne bloque pas l'entrée ultérieure du logement de verrou à l'intérieur du renfoncement (184), des cliquets (206) à l'intérieur du logement de verrou sont forcés en engagement avec des dentelures (210) découpées dans la circonférence extérieure d'un composant (212) du train de transmission, dans lequel l'engagement des cliquets (206) à l'intérieur des dentelures (210) interdit au logement de verrou (180) un mouvement de rotation et verrouille le train de transmission (14") contre un mouvement de rotation, la sélectivité permettant au chirurgien de prérégler et de verrouiller la position de la prothèse (11) avant l'installation de celle-ci, dans lequel le logement de verrou (180) peut être déverrouillé du logement de façon à permettre un démontage rapide et modulaire et à ce que le porte-prothèse soit facilement nettoyable.
